# EUROPEAN PATENT APPLICATION

(11) **EP 4 394 789 A1**
(43) Date of publication of application: **03.07.2024**
(21) Application number: 22216696.9
(22) Date of filing: 27.12.2022
(51) Int. Cl.: G16H 20/40, G16H 40/20, G16H 40/63, A61B 34/00, A61B 50/33, G16H 70/20, A61B 34/10

(54) **A METHOD FOR GENERATING A TRAY INSTRUMENT LAYOUT**

(71) Applicant: Mölnlycke Health Care AB, 402 52 Göteborg (SE)
(72) Inventor: ROSENGREN, Oscar, 436 55 HOVÅS (SE)
(74) Representative: Kransell & Wennborg KB

(57) **Abstract**

The present disclosure generally relates to a method for generating a tray instrument layout, where the tray instrument layout is adapted taking into account a type of surgical procedure to be performed, available surgical instruments and a procedural order for performing the surgical procedure. The present disclosure further relates to a corresponding computer system and computer program product.

## Description

### TECHNICAL FIELD

The present disclosure generally relates to a method for generating a tray instrument layout, where the tray instrument layout is adapted taking into account a type of surgical procedure to be performed, available surgical instruments and a procedural order for performing the surgical procedure. The present disclosure further relates to a corresponding computer system and computer program product.

### BACKGROUND

Instruments are an essential part of surgical procedures, and the correct arrangement and availability of instruments on the surgical instrument tray can greatly affect the efficiency and success of the procedure.

In a typical surgical procedure, a surgeon will employ a large number of hand-held surgical instruments in a specific sequence depending upon both the specific procedure being performed and the preference of the particular surgeon. As there are on the order of hundreds of different surgical instruments in general use, in addition to their being various sizes and other variations within these general categories, it becomes critical that the surgical assistants be able to deliver accurately the specific instrument called for by the surgeon in a rapid and highly reliable manner. Unfortunately, there are situations in the surgical environment wherein either experienced knowledgeable surgical staff make careless mistakes or assistants who are inadequately skilled are not able to identify by name the particular surgical instrument called for by a surgeon.

An example of a solution trying to counter such a situation is presented in US6158437, by providing a plastic or paper based surgical instrument support that is to be positioned within a surgical instrument tray as mentioned above. The surgical instrument support shows a graphical representation of the different surgical instruments, preferably in an expected order of use during the surgical procedure.

The surgical instrument support as presented in US6158437 is generally to be reused when the same type of surgical procedure is to be performed at a later stage in relation to another patient. To ensure that the surgical instrument tray is kept sterile, the surgical instrument support is to be placed within a transparent and sterilizable flexible pocket of the surgical instrument tray.

Even though the surgical instrument support presented in US6158437 generally allows for improvements when performing a surgical procedure, its formation is inflexible and thus unsuitable for use in a modern hospital environment, specifically when multiple surgeons and staff are sharing a common and quickly changing working environment used for different surgical procedures.

Accordingly, there appears to be desirable to allow for increased flexibility surrounding the formation of the instrument layouts to be used in relation to a surgical instrument tray, with specific focus of handling general challenges in a modern hospital environment.

### SUMMARY

According to an aspect of the present disclosure, it is therefore provided a computer implemented method for generating a tray instrument layout for a surgical procedure, wherein the method comprises receiving, at a processing unit, a request for generating the instrument layout, wherein the request comprises an indication of the surgical procedure, acquiring, using the processing unit, a desired set of surgical instruments for performing the surgical procedure, acquiring, using the processing unit, an available set of surgical instruments, forming, using the processing unit, a matching set of surgical instruments by matching the desired set of surgical instruments with the available set of surgical instruments, forming, using the processing unit and based on the surgical procedure, a sequence of the matching set of surgical instruments, acquiring, using the processing unit and based on the surgical procedure, instrument defining information for at least a portion of the matching set of surgical instruments, and forming, using the processing unit, a graphical representation of the sequence of the surgical instruments, wherein the graphical representation further comprises the instrument defining information arranged adj acent to the respective surgical instrument

The present disclosure is based upon the realization that further measures are needed for combating situations where some of the surgical instruments needed for a specific surgical procedure are not readily available, thus resulting in an all but static environment for the staff preparing for the surgical procedure. The availability of surgical instruments may as such be changing from day to day. This is a common problem in a modern hospital environment where a large number of surgical procedures are constantly performed and were a purchasing procedure for acquiring the desired surgical instruments and related inventory may not always correlate with what is currently needed.

Manually finding alternative/substitute surgical instruments to non-available surgical instruments can possibly be affected by stress, inexperienced staff and general human errors. This is line with the present disclosure solved by the introduction of an automated matching scheme, where a computerized process will ensure that the most suitable matching surgical instrument are provided and correctly position within the tray instrument layout for the surgical procedure. Advantages following this automated approach is the ability to ensure that the surgical procedure may be performed with a minimal risk for errors, even in cases with uncertain availability of the instruments to be used in the procedure.

As employed herein, the term "surgical procedure" means a procedure performed on a patient by a physician, dentist, veterinarian or other legally authorized health care professional which procedure involves a plurality of hand-held (surgical) instruments and is at least partially invasive.

The term "surgical instrument" should be treated broadly, including the above mentioned hand-held and at least partially invasive instruments, as well as swabs, sutures, etc. Possibly, also gloves, etc., may form part of the term surgical instrument, within the context of the present disclosure.

For allowing the process of matching the desired set of surgical instruments with the available set of surgical instruments to be performed with a minimum amount of perceived delay, it is desirable to apply a computational efficient matching scheme. Possible schemes that can be used in relation to the present disclosure include different forms of so-called graph-based image processing methods that today find usage within e.g. the computer vision field, such as finding paths in images. Other predefined schemes are also possible and within the scope of the present disclosure.

In case of a non-available surgical instrument, it may be possible to introduce an alternative/substitute instrument. This process is preferably performed in an automated manner by the processing unit, where the processing unit automatically make such an assessment and determination of the alternative/substitute instrument or instruments.

Possibly, the matching scheme as well as the determination of an alternative/substitute instrument may include a machine learning process for selecting the best matching instruments available, possibly taking into account the necessity of using a specific instrument for a specific surgical procedure. That is, in some surgical procedures it may be possible to allow for more flexibility in the selection of an alternative instrument, as compared to other surgical procedures where some instruments are of absolute necessity. The machine learning process may in such an embodiment be trained to also take into account a current status of an inventory of instruments. Further expected (future) surgical procedures may also be taken into account in determining the most suitable matching instrument.

The machine learning process may for example be one of an unsupervised machine learning process or a supervised machine learning process. Using a supervised machine learning process may be initially advantageous (when in comparison a smaller amount of data is available), whereby the result of the machine learning process may be reviewed by a person, such as the surgeon or staff mentioned above, for determining if the outcome of the machine learning process is likely correct.

Different types of machine learning processes may be used, such as for example a machine learning process based on a convolutional neural network (CNN) or a recurrent neural network (RNN).

In accordance with an embodiment of the present disclosure it may be desirable to produce a physical copy of the graphical representation. The physical copy of the graphical representation may for example be printed onto a sheet of material, such as manufactured from paper or plastic. It is desirable to select the sterilization method, the printing method and/or the sheet of material to be able to be sterilized without affecting the graphical representation or the sheet of material in itself. In case the sheet of material is from paper, it may for example be possible to use a sterilization method that is based on gamma irradiation or dry heat, to ensure that the paper survive the sterilization. The printing method used may for example include laser jet printing or inkjet printing, while other methods also are possible within the scope of the present disclosure.

In some embodiments it may be possible to allow the instrument defining information to comprise a computer readable element. In one embodiment, the computer readable element is optically readable using the electronic device, for example implemented as at least one of a one-dimensional (1D) and a two-dimensional (2D) bar code. Other optical methods, present and future, may be possible and are within the scope of the present disclosure. In case of using an optically computer readable element, possibly a so called QR code may be used.

An advantage of including a computer readable element with at least a portion of the surgical instruments is the ability of allowing a surgeon or staff to easily acquire further information about the surgical instrument and its use. For example, the surgeon or staff may use an electronic device equipped with e.g. a camera or similar means for capturing the optically computer readable element. The electronic device may for example be a mobile phone or similar. The optically computer readable element may include a hyperlink or similar to a website (or similar) that displays information (within a GUI of the electronic device), including images and video sequences, further defining the surgical instrument and its use.

In a preferred embodiment of the present disclosure the request further comprises an additional set of surgical instruments, and the matching set of surgical instruments is further based on the additional set of surgical instruments. Accordingly, a specific surgeon may have a personally desirable instrument to use in relation to a specific surgical procedure, in addition to what is normally expected to be used in a specific surgical proceedure. The process according to the present disclosure ensures that also such requirements are included when preparing the graphical representation of the surgical instruments.

In some embodiments of the present disclosure, it may be desirable to further allow the method to comprise establishing, using the processing unit, a connection with a server storing graphical illustrations of surgical instruments, and requesting, using the processing unit, a graphical illustration for each of the surgical instruments in the sequence of the surgical instruments to be comprised in the graphical representation. Advantageously, the graphical illustrations will further simplify the task of preparing the tray for the surgical procedure, since the staff preparing the task now will be provided with an illustration matching each of the instruments. As an alternative, the graphical illustration may solely be provided as an outline of the corresponding surgical instrument.

It should be understood that the server preferably comprises the above discussed processing unit. The computational power provided by the processing unit and/or the server could for example be provided in the form of a cloud-based computing system, where the server is defined as a cloud server. Thus, the computing power provided by means of the present may be distributed between a plurality of servers, and the location of the servers must not be explicitly defined. Advantageous following the use of a cloud-based solution is also the inherent redundancy achieved.

Possibly, the method may further comprise uploading, using the processing unit, an image of a further surgical instrument to the server, and automatically forming, using the server, an outline of the further surgical instrument. Accordingly, it may be possible to take one or a plurality of photos of further/new instruments, whereby the server automatically will apply an image processing scheme for extracting an instrument outline. Possibly, the above discussed mobile phone may be used for capturing the image(s) of the further/new instruments.

According to another aspect of the present disclosure, there is further provided a computer system adapted to generate a tray instrument layout for a surgical procedure, wherein the computer system comprises a processing unit adapted to receive a request for generating the instrument layout, wherein the request comprises an indication of the surgical procedure, acquire a desired set of surgical instruments for performing the surgical procedure, acquire an available set of surgical instruments, form a matching set of surgical instruments by matching the desired set of surgical instruments with the available set of surgical instruments, form, based on the surgical procedure, a sequence of the matching set of surgical instruments, acquire, based on the surgical procedure, instrument defining information for at least a portion of the matching set of surgical instruments, and form a graphical representation of the sequence of the surgical instruments, wherein the graphical representation further comprises the instrument defining information arranged adjacent to the respective surgical instrument. This aspect of the invention provides similar advantages as discussed above in relation to the previous aspects of the invention.

Advantageously, the computer system further comprises a network interface for communication with an electronic user device, and the electronic user device is adapted to form the request for generating the instrument layout. As mentioned above, the electronic user device may for example be a mobile phone. However, any form of mobile electronic device is possible, such as a computer (e.g. laptop), preferably provided with the above discussed camera for acquiring an image or video sequence.

According to a further aspect of the present invention, there is provided a program product comprising a non-transitory computer readable medium having stored thereon computer program means for controlling a computer system adapted to generate a tray instrument layout for a surgical procedure, wherein the computer system comprises a processing unit and the computer program product comprises code for receiving, at a processing unit, a request for generating the instrument layout, wherein the request comprises an indication of the surgical procedure, code for acquiring, using the processing unit, a desired set of surgical instruments for performing the surgical procedure, code for acquiring, using the processing unit, an available set of surgical instruments, code for forming, using the processing unit, a matching set of surgical instruments by matching the desired set of surgical instruments with the available set of surgical instruments, code for forming, using the processing unit and based on the surgical procedure, a sequence of the matching set of surgical instruments, code for acquiring, using the processing unit and based on the surgical procedure, instrument defining information for at least a portion of the matching set of surgical instruments, and code for forming, using the processing unit, a graphical representation of the sequence of the surgical instruments, wherein the graphical representation further comprises the instrument defining information arranged adjacent to the respective surgical instrument.

A software executed by the processing unit for operation in accordance to the invention may be stored on a computer readable medium, being any type of memory device, including one of a removable nonvolatile random access memory, a hard disk drive, a floppy disk, a CD-ROM, a DVD-ROM, a USB memory, an SD memory card, or a similar computer readable medium known in the art.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Fig. 1 conceptually shows a computer system according to a currently preferred embodiment of the present disclosure adapted for forming a physical copy of a tray instrument layout,
Fig. 2 discloses an exemplary a graphical representation of a sequence of surgical instruments of the tray instrument layout, and
Fig. 3 is a flow chart illustrating the steps of performing the method according to a currently preferred embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

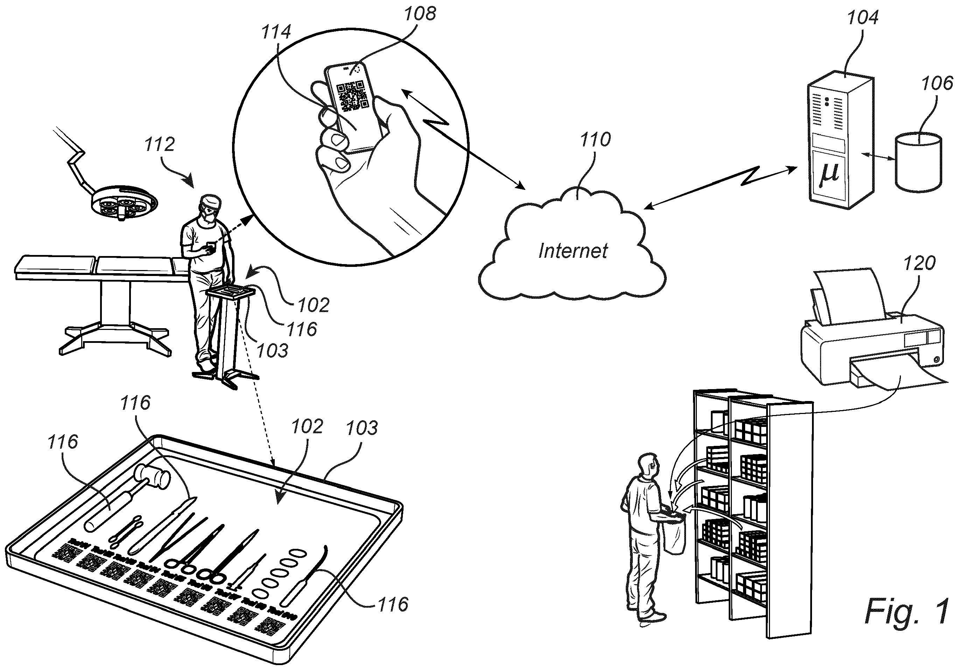

Turning now to the drawings and to Fig. 1 in particular, there is conceptually illustrated a computer system 100 adapted for adapted to generate a tray instrument layout 102 for a surgical procedure to be arranged at a surgical instrument tray 103.

The computer system 100 comprises a server 104 arranged in communication with a database 106, and a client device 108 arranged in networked communication, such as using the Internet 110, with the server 104. In Fig. 1, the client device 108 is operated by a hospital staff member 112. However, any user, such as for example a surgeon, may be allowed to operate the client device 108.

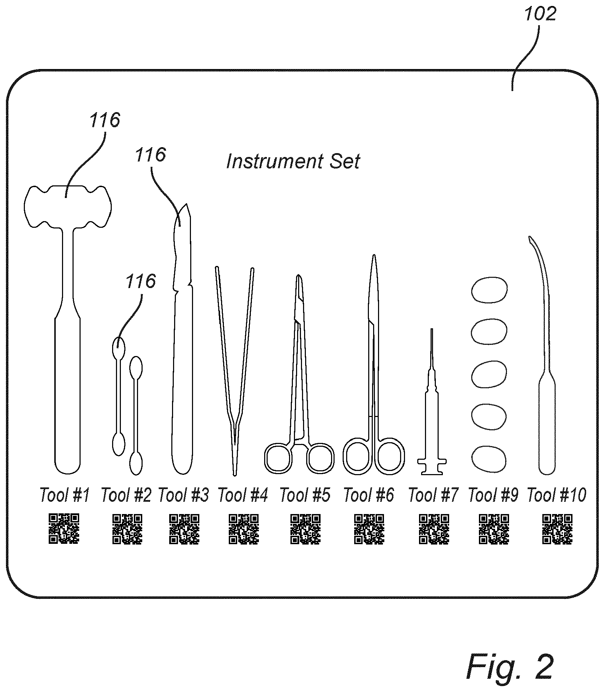

The client device 108, is here illustrated as a mobile phone, comprises a graphical user interface (GUI) 114 and a camera (not explicitly shown). The client device 108 further comprises a control unit (not shown). The GUI 114 is preferably adapted to present instructions and information to e.g. the staff member 112, such as for acquiring images of a computer readable elements 202 (as will be further illustrated in Fig. 2 and elaborated below) as well as of surgical instruments 116 using the camera (not shown) and for displaying information in relation to the surgical instruments 116.

The network 110 may be wired or wireless, including for example wired connections like a building LAN, a WAN, an Ethernet network, an IP network, etc., and wireless connections like WLAN, CDMA, GSM, GPRS, 3G mobile communications, 4G mobile communications, 5G mobile communications, Bluetooth, infrared, or similar.

The computer system 100 further comprises a printer 118, preferably also provided in networked connection with the server 104.

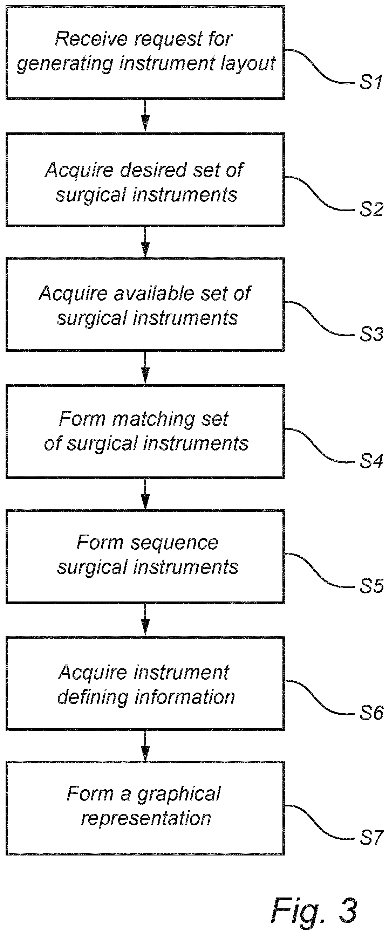

During operation of the computer system 100, with further reference to Figs. 2 and 3, the process starts with the server 104 receiving, S1, a request for generating the instrument layout. The request for generating the instrument layout may normally form part of a request for a set of surgical instruments 116 to be used in a specific surgical procedure. As such, the request for generating the instrument layout comprises an indication, such as a data tag, holding information about the specific surgical procedure. The surgical procedure can be any form of surgical procedure where surgical instruments 118 are to be used.

The server 104 subsequently acquires, S2, a desired set of surgical instruments 118 for performing the specific surgical procedure. As an example, the server 102 may request information from e.g. the database 106 based on the surgical procedure and as a response receive a list of the desired set of surgical instruments 118 that are to be used for performing the specific surgical procedure.

The server 104 will then proceed to acquire, S3, e.g. a list of available surgical instruments. Such a list may for example be stored by the database 106 and reflect a current inventory of surgical instrument, such as within a hospital, at an external party warehouse, etc.

Based on the list of the desired set of surgical instruments 118 and the list of available surgical instruments the server 104 will proceed to form, S4, a matching set of surgical instruments. As will be apparent from the above, it may be situations where not all of the desired surgical instruments 118 are available. In such a case it may be possible to introduce a substitute or alternative instrument or instruments. The server 104 may in possibly be configured to automatically make such an assessment and determination of the substitute or alternative instrument or instruments. Possibly, the server 104 may in such an embodiment take into account other, possibly parallel, requests for surgical instrument.

The server 104 may possibly implement a strategy where critical surgical procedures will be provided with the desired surgical instruments, and less critical surgical procedures will be provided with alternative/substitute instruments. Other strategies may of course be possible and within the scope of the present disclosure.

Additionally, the server 104 could possibly take into account an expected change in the inventory of surgical instruments 118. That is, in case the server 104 received an indication that there is expected an intermediate interruption in the inventory, the server 104 may select to automatically make use of alternative/substitute instruments. It should be understood that the server 104 may make use of a machine learning process for performing the above discussed strategies for selecting alternative/substitute instruments. Such a machine learning process may possibly be trained based on previous human actions, where a human operator previously made considerations for compiling the matching set of surgical instruments.

Once a matching set of surgical instruments has been formed, the server 104 may proceed to form, S5, a sequence of the matching set of surgical instruments. The server 104 may in such a situation acquire information from e.g. the database 106 that presents an general order for the specific surgical procedure. The server 104 may then amend the order based on the matching set of surgical instruments. That is, since the matching set of surgical instruments may differ from the desired set of instruments for the surgical procedure, such an amendment may be needed.

It will generally be desirable to acquire instrument defining information for at least a portion of the matching set of surgical instruments, where the instrument defining information comprises the above-mentioned computer readable element 202. The computer readable element 202 may, as exemplified in Fig. 2, include a so called QR code. The QR code may in itself hold information relating to a surgical instrument or comprise a reference to an online service (such as a website) where information may be found that relates to the function and operation of the surgical instrument.

It may additionally be desirable to request a graphical illustration for each of the surgical instruments in the sequence of the matching set of surgical instruments 118. Such a graphical illustration may for example comprises an outline 204 of the corresponding surgical instrument 118. Alternatively, the graphical illustration may be an image of the instrument 118.

Once the server has the sequence of the matching set of surgical instruments 118, the computer readable element 202 and the outlines 204 available, the server 104 will proceed to form, S7, the graphical representation of the sequence of the surgical instruments 118. The graphical representation may be seen as holding all the above listed information, arranged in a suitable manner, and taking into account a maximum length/with allowed and the size of each of the instruments 118. The server 104 is preferably arranged to automatically arrange the instruments 118 according to the formed sequence, where the QR code 202 (or similar) is arranged adjacent to the respective surgical instrument 118.

The graphical representation may then be transmitted to the printer 118, where the printer may print the graphical representation onto e.g. a sheet of paper, thereby forming the tray instrument layout 102, to be arranged at the surgical instrument tray 103.

In a warehouse or storage facility it may be possible to package the tray instrument layout 102 together with the matching set of instruments 118 for the surgical procedure, e.g. in a container or "bag". The container/bag may then be unpacked in an operating theater by the staff member 112, where the staff member 112 will arrange the tray instrument layout 102 at the surgical instrument tray 103 and then arrange the surgical instruments 118 aligned with the outlined of each of the instruments 118. In case the staff member 112 is in need of further information for a specific instrument 118, the staff member 112 may capture an image of the QR code 202 and be presented with further information within the GUI 114 of the mobile phone 108.

The control functionality of the present disclosure may be implemented using existing computer processors, or by a special purpose computer processor for an appropriate system, incorporated for this or another purpose, or by a hardwire system. Embodiments within the scope of the present disclosure include program products comprising machine-readable medium for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage or other magnetic storage devices, or any other medium which can be used to carry or store desired program code in the form of machine-executable instructions or data structures, and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general-purpose computer, special purpose computer, or special purpose processing machines to perform a certain function or group of functions.

Although the figures may show a sequence the order of the steps may differ from what is depicted. Also two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule-based logic and other logic to accomplish the various connection steps, processing steps, comparison steps and decision steps. Additionally, even though the invention has been described with reference to specific exemplifying embodiments thereof, many different alterations, modifications and the like will become apparent for those skilled in the art.

In addition, variations to the disclosed embodiments can be understood and effected by the skilled addressee in practicing the claimed invention, from a study of the drawings, the disclosure, and the appended claims. Furthermore, in the claims, the word "comprising" does not exclude other elements or steps, and the indefinite article "a" or "an" does not exclude a plurality.

## Claims

1. A computer implemented method for generating a tray instrument layout (102) for a surgical procedure, wherein the method comprises:
- receiving (S1), at a processing unit, a request for generating the instrument layout, wherein the request comprises an indication of the surgical procedure,
- acquiring (S2), using the processing unit, a desired set of surgical instruments (118) for performing the surgical procedure,
- acquiring (S3), using the processing unit, an available set of surgical instruments,
- forming (S4), using the processing unit, a matching set of surgical instruments by matching the desired set of surgical instruments with the available set of surgical instruments,
- forming (S5), using the processing unit and based on the surgical procedure, a sequence of the matching set of surgical instruments,
- acquiring (S6), using the processing unit and based on the surgical procedure, instrument defining information for at least a portion of the matching set of surgical instruments, and
- forming (S7), using the processing unit, a graphical representation of the sequence of the surgical instruments, wherein the graphical representation further comprises the instrument defining information arranged adj acent to the respective surgical instrument.

2. The method according to claim 1, further comprising:
- producing a physical copy of the graphical representation.

3. The method according to any one of claims 1 and 2, wherein the instrument defining information comprises a computer readable element (202).

4. The method according to any one of the preceding claims, wherein the request further comprises an additional set of surgical instruments, and the matching set of surgical instruments is further based on the additional set of surgical instruments.

5. The method according to any one of the preceding claims, further comprising:
- identifying, using the processing unit, an alternative surgical instrument if a desired surgical instrument is not matched with an available surgical instrument.

6. The method according to any one of the preceding claims, further comprising:
- establishing, using the processing unit, a connection with a server (104) storing graphical illustrations of surgical instruments, and
- requesting, using the processing unit, a graphical illustration for each of the surgical instruments in the sequence of the surgical instruments to be comprised in the graphical representation.

7. The method according to claim 6, wherein each graphical illustration comprises an outline of the corresponding surgical instrument.

8. The method according to any one of claims 6 and 7, further comprising:
- uploading, using the processing unit, an image of a further surgical instrument to the server, and
- automatically forming, using the server, an outline of the further surgical instrument.

9. A computer system adapted to generate a tray instrument layout for a surgical procedure, wherein the computer system comprises a processing unit adapted to:
- receive a request for generating the instrument layout, wherein the request comprises an indication of the surgical procedure,
- acquire a desired set of surgical instruments for performing the surgical procedure,
- acquire an available set of surgical instruments,
- form a matching set of surgical instruments by matching the desired set of surgical instruments with the available set of surgical instruments,
- form, based on the surgical procedure, a sequence of the matching set of surgical instruments,
- acquire, based on the surgical procedure, instrument defining information for at least a portion of the matching set of surgical instruments, and
- form a graphical representation of the sequence of the surgical instruments, wherein the graphical representation further comprises the instrument defining information arranged adjacent to the respective surgical instrument.

10. The computer system according to claim 9, wherein:
- the system further comprises a network interface for communication with an electronic user device, and
- the electronic user device is adapted to form the request for generating the instrument layout.

11. The computer system according to claim 10, wherein the system is adapted to establish a communication with a printer, using the network interface, for producing a physical copy of the graphical representation.

12. The computer system according to any one of claims 10 and 11, wherein the request further comprises an additional (personal) set of surgical instruments, and the matching set of surgical instruments is further based on the additional set of surgical instruments.

13. The computer system according to any one of claims 9 - 12, wherein the processing unit is further adapted to:
- identify an alternative surgical instrument if a desired surgical instrument is not matched with an available surgical instrument.

14. The computer system according to any one of claims 10 - 11, wherein the processing unit is further adapted to:
- establish, using the network interface, a connection with a server storing graphical illustrations of surgical instruments, and
- request a graphical illustration for each of the surgical instruments in the sequence of the surgical instruments to be comprised in the graphical representation.

15. A computer program product comprising a non-transitory computer readable medium having stored thereon computer program means for controlling a computer system adapted to generate a tray instrument layout for a surgical procedure, wherein the computer system comprises a processing unit and the computer program product comprises:
- code for receiving, at a processing unit, a request for generating the instrument layout, wherein the request comprises an indication of the surgical procedure,
- code for acquiring, using the processing unit, a desired set of surgical instruments for performing the surgical procedure,
- code for acquiring, using the processing unit, an available set of surgical instruments,
- code for forming, using the processing unit, a matching set of surgical instruments by matching the desired set of surgical instruments with the available set of surgical instruments,
- code for forming, using the processing unit and based on the surgical procedure, a sequence of the matching set of surgical instruments,
- code for acquiring, using the processing unit and based on the surgical procedure, instrument defining information for at least a portion of the matching set of surgical instruments, and
- code for forming, using the processing unit, a graphical representation of the sequence of the surgical instruments, wherein the graphical representation further comprises the instrument defining information arranged adj acent to the respective surgical instrument.
